# EUROPÄISCHE PATENTSCHRIFT

(11) **EP 2 326 389 B1**
(45) Veröffentlichungstag und Bekanntmachung des Hinweises auf die Patenterteilung: **25.01.2017**
(21) Anmeldenummer: 09778501.8
(22) Anmeldetag: 12.09.2009
(51) Int. Cl.: A61N 5/10

(54) **SCHNELLES SCANNING EINES ZIELGEBIETS**
FAST SCANNING OF A TARGET REGION
BALAYAGE RAPIDE D'UNE ZONE CIBLE

(30) Priorität: 26.09.2008 DE 102008048916
(43) Veröffentlichungstag der Anmeldung: 01.06.2011
(73) Patentinhaber: GSI Helmholtzzentrum für Schwerionenforschung GmbH, 64291 Darmstadt (DE); Siemens Aktiengesellschaft, 80333 München (DE); Heidelberger Ionenstrahltherapie (Hit) Betriebs-Gesellschaft Am Universitätsklinikum Heidelberg MbH, 69120 Heidelberg (DE)
(72) Erfinder: BERT, Christoph, 63741 Aschaffenburg (DE); HABERER, Thomas, 60528 Frankfurt (DE); RIETZEL, Eike, 64331 Weiterstadt (DE)
(74) Vertreter: Mergel, Volker
(86) Internationale Anmeldenummer: PCT/EP2009/006626
(87) Internationale Veröffentlichungsnummer: WO 2010/034419

(56) Entgegenhaltungen:
- EP-A1- 0 986 070
- EP-A1- 1 041 579
- EP-A1- 1 045 399
- EP-A2- 1 905 481
- WO-A1-02/41948
- DE-A1- 19 907 097
- BERT CHRISTOPH: "Bestrahlungsplanung für bewegte Zielvolumina in der Tumortherapie mit gescanntem Kohlenstoffstrahl" INTERNET CITATION, [Online] 3. Februar 2006 (2006-02-03), XP002481370 Gefunden im Internet: URL:http://elib.tu-darmstadt.de/diss/00064 8> [gefunden am 2008-06-24]
- HABERER T ET AL: "MAGNETIC SCANNING SYSTEM FOR HEAVY ION THERAPY" NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, Bd. A330, Nr. 1/02, 10. Juni 1993 (1993-06-10), Seiten 296-305, XP000385496 ISSN: 0168-9002 in der Anmeldung erwähnt
- Anonymous: "Steuerungstechnik" Wikipedia, der freien Enzyclopädie, [Online] XP002560594 Gefunden im Internet: URL:http://de.wikipedia.org/wiki/Steuerung stechnik> [gefunden am 2009-12-15]

## Beschreibung

Die vorliegende Erfindung bezieht sich auf ein Verfahren und eine Vorrichtung zum Bestrahlen eines Ziels mit einem Zielpunkte anfahrenden Strahl, sowie auf ein Kontrollsystem zur Kontrolle der erfindungsgemäßen Vorrichtung.

Die Bestrahlung eines Ziels mit einem verschiedene Punkte anfahrenden Strahl (Beam Scanning) ist an sich bekannt. So werden etwa bei der Bestrahlung von Tumoren beispielsweise Partikelstrahlen, vor allem Ionenstrahlen, insbesondere mit Protonen, α-Teilchen oder Kohlenstoffkemen, eingesetzt. Es werden Teile des Zielgebiets, die Zielpunkte, sequenziell nacheinander mit dem Strahl angefahren.

Partikelstrahlen sind für die Bestrahlung von Zielvolumina besonders vorteilhaft, da sie zu ihrem Ende hin ein Maximum in der Energiedeposition durchlaufen (Bragg-Peak). So können etwa auch eingebettete räumliche Strukturen effektiv bestrahlt werden, ohne die einbettende Umgebung allzu stark zu schädigen. Oft werden räumliche Zielgebiete schichtweise bestrahlt, wobei die die Eindringtiefe bestimmende Strahlenergie je Schicht konstant gewählt wird (Isoenergieschicht). Bekannt ist auch das so genannte volumetrische Scanning, bei dem die hintereinander angefahrenen Zielpunkte nicht notwendig einzelnen (Isoenergie-)Schichten zugeordnet sind. Die Erfindung betrifft grundsätzlich auch Ausführungsformen, bei denen der Strahl durch elektromagnetische Wellen gebildet wird. Weiter betrifft die Erfindung grundsätzlich auch Ausführungsformen zur Bestrahlung eines flächigen Zielgebiets.

Scanning-Verfahren ermöglichen eine an die Form des Ziels angepasste Bestrahlung durch Abtasten mit einem Strahl; es werden verschiedene Scanning-Verfahren unterschieden.

Bei dem so genannten Spotscanverfahren und dem Pixelscanverfahren verweilt der Partikelstrahl an jedem Zielpunkt für eine vorbestimmte Zeit und/oder deponiert an jedem Zielpunkt eine vorbestimmte Anzahl Partikel und wird ausgeschaltet, während Ablenkmagnete (Scanning-Magnete) auf einen nächsten Zielpunkt eingestellt werden.

Bei dem so genannten Rasterscanning verweilt der Partikelstrahl an jedem von vielen Rasterpunkten während einer vorbestimmten Zeitdauer oder deponiert an jedem Rasterpunkt eine vorbestimmte Anzahl Partikel, wird aber zwischen den Rasterpunkten nicht oder nicht immer ausgeschaltet. Die Rasterpunkte können sich von den Zielpunkten unterscheiden, da die Rasterpunkte i.d.R. in dem Koordinatensystems der Bestrahlungsvorrichtung angegeben werden, während die Zielpunkte i.d.R. in dem Koordinatensystem des Ziels betrachtet werden, insbesondere müssen Rasterpunkte und Zielpunkte nicht deckungsgleich übereinander liegen, obwohl dies bevorzugt ist. Der sprachlichen Einfachheit halber werden unten die beim Rasterscanning angefahrenen Punkte nicht durchgängig als Rasterpunkte, sondern auch als Zielpunkte bezeichnet.

Bei so genannten kontinuierlichen Scanning-Verfahren bilden die Zielpunkte zusammenhängende Linien, bilden also kontinuierliche (oder quasikontinuierliche) Mengen, wobei ihre Anzahl etwa abzählbar unendlich sein kann. Der Partikelstrahl wird bei einem kontinuierlichen Scanning-Verfahren zumindest innerhalb einer Linie bzw. Zeile in einer Isoenergieschicht kontinuierlich abgelenkt und überstreicht die Zielpunkte, ohne an einzelnen Orten zu verweilen. Mit einer Tiefenmodulationsvorrichtung kann auch ein kontinuierliches Scanning-Verfahren durchgeführt werden, bei dem kontinuierlich die Eindringtiefe des Partikelstrahls moduliert wird.

Es ist auch sogenanntes "uniform scanning" bekannt, bei dem jeweils in einer Isoenergieschicht eine homogene Dosisdeposition erzielt wird, indem einmal oder aber auch mehrfach die gleiche Teilchenzahl an allen Zielpunkten bzw. über die Scanpfade deponiert wird.

Grundsätzlich kann die Abfolge der Zielpunkte, der Pfad, etwa innerhalb einer isoenergetischen Schicht verlaufen, indem der Strahl lediglich in seiner Verlaufsrichtung, also ein- oder zweidimensional lateral, abgelenkt wird, oder er kann etwa auch zwischen isoenergetischen Schichten verlaufen, indem die Energie des Strahls geändert wird.

Es ist an sich bekannt, die Position des Strahls beziehungsweise dessen Lage im Raum während der Bestrahlung zu messen. Ebenso ist es bekannt, auch die Intensität, etwa als Teilchenfluss oder applizierte Teilchenzahl, des Strahls während der Bestrahlung zu messen. Der Begriff Intensität wird hier für alle entsprechenden Größen der sprachlichen Einfachheit halber einheitlich verwendet.

Die Publikation "Magnetic scanning system for heavy ion therapy", Haberer et al., Nuclear Instruments and Methods in Physics Research A330 (1993) 296 ff. zeigt etwa eine Vieldraht-Proportionalkammer (MWPC; Multi-Wire-Proportional-Chamber), um während der Bestrahlung der Position des Strahls zu messen. Weiter ist es hier gezeigt, die Intensität des Strahls zu messen und das Ergebnis der Messung zur Steuerung der Bestrahlung zu verwenden (Intensitätssteuerung). So wird der Strahl etwa schneller bewegt, wenn die zu deponierende Dosis pro Zielpunkt relativ gering und die Strahlintensität hoch ist; er wird entsprechend langsamer bewegt, wenn die zu deponierende Dosis pro Zielpunkt relativ hoch und die Strahlintensität gering ist. In anderen Worten: Der Strahl wird auf den jeweils nächsten Zielpunkt gerichtet, wenn die erforderliche Teilchenzahl an dem vorangehenden Zielpunkt deponiert ist.

Grundsätzlich ist es in Fachkreisen auch bekannt, die gemessene Position des Strahls und dessen gemessene Intensität zu verwenden, um diese jeweils während der Bestrahlung zu korrigieren, falls Abweichungen von Sollwerten auftreten. Gegebenenfalls wird die Bestrahlung auch unterbrochen, etwa bei einer größeren Abweichung vom Soll. Bisher werden, um eine hohe Genauigkeit der Messungen zu erreichen, diese mehrfach pro Zielpunkt durchgeführt. Dies gilt sowohl für die Position des Strahls als auch für dessen Intensität. Zusätzlich kann auch die Strahlbreite gemessen werden.

Um genug Zeit für die Positionsmessungen zu haben, ist es bekannt, die Strahlintensität so anzupassen, dass für jeden Zielpunkt die Messungen durchgeführt werden können. Oftmals wird die Strahlintensität dazu geringer gewählt als vom Beschleuniger her möglich.

Der vorliegenden Erfindung liegt die Aufgabe zu Grunde, ein vorteilhaftes Verfahren und eine vorteilhafte Vorrichtung zum Bestrahlen eines Ziels mit einem Zielpunkte anfahrenden Strahl, sowie ein Kontrollsystem zur Kontrolle der erfindungsgemäßen Vorrichtung anzugeben.

Die Aufgabe wird gelöst durch ein Verfahren zum Bestrahlen eines Ziels mit einem Zielpunkte anfahrenden Strahl mit den Schritten: Messen zumindest einer der Größen Position des Strahls und Intensität des Strahls, Ändern des Strahls in Abhängigkeit von der zumindest einen gemessenen Größe, insbesondere von einer Abweichung betreffend die zumindest eine gemessene Größe. Das Verfahren ist dadurch gekennzeichnet, dass pro Zielpunkt die zumindest eine gemessene Größe höchstens einmal gemessen wird.

Bevorzugte Ausgestaltungen der Erfindung sind in abhängigen Ansprüchen angegeben und werden im Folgenden näher erläutert.

Die Erfindung beruht auf der Feststellung, dass sowohl die Messung der Position des Strahls als auch die Messung der Intensität des Strahls eine minimale Zeit benötigen. Sie beruht weiter auf der Erkenntnis, dass die oben beschriebenen Messzyklen die Scangeschwindigkeit begrenzen.

Vor allem wenn die Strahlintensität so angepasst wird, dass mehrere Positionsmessungen pro Zielpunkt durchgeführt werden können, kann die gesamte Bestrahlungszeit recht lang sein, insbesondere falls dann das Ziel auch noch in mehreren Durchgängen angefahren wird (Rescanning, s.u.); auch wirkt sich die vergleichsweise geringe Intensität negativ auf das Signal-zu-Rausch-Verhältnis der Intensitätsmessung aus.

Weiter wurde festgestellt, dass die durch die mehrfache Messung erreichbare Genauigkeit bezüglich der Strahlposition und/oder der Strahlintensität bzw. die Kenntnis der Strahlbreite nicht zwingend notwendig ist, etwa weil die Vorrichtung für eine entsprechend exakte Strahlführung ausgelegt oder weil aufgrund einer speziellen Ausgestaltung der Bestrahlung diese besonders fehlertolerant ist (vgl. Rescanning, s.u.).

Idee der Erfindung ist es, die jeweils zu messende Größe, also die Position des Strahls und/oder die Intensität des Strahls, pro Zielpunkt höchstens einmal zu messen. Die Erfindung umfasst den Fall, dass lediglich nur eine dieser beiden Größen höchstens einmal pro Zielpunkt gemessen wird. Sie umfasst insbesondere auch die Fälle, dass eine der beiden Größen mehrmals pro Zielpunkt oder gar nicht bzw. verteilt über mehrere Zielpunkte gemessen wird und die jeweils andere Größe lediglich höchstens einmal pro Zielpunkt.

Die aktuelle Technologie ermöglicht deutlich schnellere Intensitätsmessungen als Positionsmessungen. Bei einer bevorzugten Ausführungsform wird entsprechend die Positionsmessung höchstens einmal pro Zielpunkt durchgeführt, während die Intensitätsmessung durchaus mehrfach pro Zielpunkt durchgeführt wird.

Insbesondere ist es bevorzugt, die Scangeschwindigkeit basierend auf der gemessenen Intensität des Strahls zu bestimmen (vgl. Intensitätssteuerung; s.o.) und dazu die Intensität des Strahls gegebenenfalls mehrfach pro Zielpunkt zu messen, während die Position des Strahls höchstens einmal pro Zielpunkt bestimmt wird.

Die Positions- und/oder Intensitätsmessungen können dazu genutzt werden, eine Abweichung von vorgegebenen Werten bzw. Sollwerten, man kann auch Fehler sagen, festzustellen. An eine solche Feststellung können Änderungen des Strahls geknüpft sein. Im einfachsten Fall wird die Bestrahlung unterbrochen oder sogar abgebrochen, wenn das Ergebnis der Positionsmessung und/oder das Ergebnis der Intensitätsmessung eine bestimmte Abweichung überschreiten. Im Rahmen der Erfindung ist es auch denkbar, die Position und/oder die Intensität des Strahls abhängig von der Größe der Abweichung zu konigieren.

Besonders bevorzugt ist es, die Messung einer der beiden Größen oder sogar beider Größen lediglich zur Überwachung des Strahls einzusetzen und bei Überschreiten einer Abweichung die Bestrahlung zu unterbrechen. Bei dieser bevorzugten Ausführungsform werden die Messungen nicht zur Korrektur des Strahls eingesetzt. So kann etwa auch die Zeit zur Korrektur der Einstellung der Scanning-Magnete (s. Beispiel) während der Bestrahlung eingespart werden.

Neben der Bestrahlung von Personen oder Tieren ist auch die Bestrahlung von organischem Material überhaupt, insbesondere von Zellen, oder auch die Bestrahlung anorganischer Materialien, etwa von Kunststoffen, relevant; etwa im Rahmen der Materialforschung.

Vorzugsweise wird Rasterscanning eingesetzt, dies ermöglicht ein besonders schnelles Scannen.

Bei der an sich bekannten Mehrfachbestrahlung mit einem verschiedene Zielpunkte anfahrenden Strahl (Rescanning) wird eine Solldosis während einer Sitzung in mehreren, gegebenenfalls durch kurze Pausen getrennten, Durchgängen (Scans) hintereinander appliziert. Innerhalb der einzelnen Durchgänge werden dabei mehrere der Zielpunkte, nicht notwendigerweise alle, sequenziell bestrahlt. Im Laufe einer Sitzung wird üblicherweise ein Großteil der Zielpunkte beziehungsweise werden sämtliche Zielpunkte und damit ein Großteil des Zielgebiets beziehungsweise das gesamte Zielgebiet mehrfach bestrahlt. Die für die Sitzung zu applizierende Gesamtdosis pro Zielpunkt wird auf die einzelnen Durchgänge aufgeteilt, und zwar etwa gleichverteilt oder gegebenenfalls auch mit unterschiedlicher Gewichtung. Auch die Mehrfachbestrahlung kann schichtweise oder volumetrisch erfolgen.

Gerade für Rescanning ist eine hohe Scangeschwindigkeit vorteilhaft, da durch die Aufteilung in mehrere Durchgänge der pro Sitzung zurückzulegende Pfad deutlich länger ist als bei einer Deposition der gesamten Dosis in einem Durchgang. Ohne die Erfindung kann die Bestrahlungsdauer insgesamt zu lang sein, etwa weil die Bestrahlung zu belastend für eine zu bestrahlende Person ist; eine Immobilisierung verschlechtert sich mit der Zeit; der Durchsatz der Anlage bleibt vergleichsweise gering.

Rescanning erlaubt den Einsatz der Erfindung auch bei weniger genau arbeitenden Anlagen, da Ungenauigkeiten in der Strahlposition und in der Strahlintensität durch Mitteln über mehrere Durchgänge klein gehalten werden können. Bei entsprechender Auslegung der Mehrfachbestrahlung, ist eine genaue Kenntnis der Strahlposition beziehungsweise der Strahlintensität während der Bestrahlung nicht erforderlich.

Können Intensitätsmessungen schneller als Positionsmessungen durchgeführt werden, so bietet es sich auch im Rahmen des Rescanning an, den Strahl über seine Intensität zu steuern und Positionsmessungen höchstens einmal pro Zielpunkt durchzuführen.

Wird ein bewegtes Ziel bestrahlt, so ist es vorteilhaft, Rescanning einzusetzen. Durch die Bewegung des Ziels kommt es nämlich zu Abweichungen von der Solldosisverteilung; auch etwa durch eine Interferenz zwischen dem Ablauf der Bestrahlung und der Bewegung des Ziels. Wird in mehreren Durchgängen bestrahlt, so können sich diese Fehldosierungen ausmitteln. Im Rahmen einer Partikeltherapie kann so ein bewegtes Ziel etwa sein, ein Tumor in der Nähe der Lunge einer zu bestrahlenden Person, welcher durch die Atmung zyklisch auf und ab bewegt wird.

Vorzugsweise wird die zumindest eine gemessene Größe höchstens einmal für zumindest zwei Zielpunkte gemessen; zunehmend bevorzugt in der angegebenen Reihenfolge höchstens einmal für drei Zielpunkte, 10 Zielpunkte oder eine Zeile Zielpunkte. Besonders bevorzugt ist es, die Anzahl der Messungen so gering zu halten, dass die für die Messungen notwendige Zeit nicht mehr die Scangeschwindigkeit begrenzt. Dies kann etwa bei einer intensitätsgesteuerten Bestrahlung der Fall sein, wenn die Zeit, die notwendig ist, um die vorgesehene Dosis in mehreren Zielpunkten zu applizieren, die Zeit für die Positionsmessung über diese Zielpunkte überschreitet.

Wird eine Messung für mehrere Zielpunkte höchstens einmal ausgeführt und dauert die Messung länger als die Bestrahlung der einzelnen Zielpunkte, so ergibt sich ein über mehrere Zielpunkte gemittelter Wert. So wird die Strahlintensität etwa bei einer intensitätsgesteuerten Bestrahlung i.d.R. von Zielpunkt zu Zielpunkt unterschiedlich ausfallen, insgesamt also eine inhomogene Verteilung vorliegen.

Um einen möglichst aussagekräftigen Mittelwert, insbesondere betreffend Positionsmessungen und gegebenenfalls Strahlbreitemessungen, zu erhalten, kann es vorteilhaft sein, auch zeitliche Effekte, wie z.B. Detektoreigenschaften, etwa die Ausbreitung der Ionisation im Detektorgas, zu berücksichtigen. Dies kann speziell für eine präzise Korrektur der Strahlposition während der Bestrahlung hilfreich sein.

Bei einer bevorzugten Ausführungsform der Erfindung werden die Zeitpunkte der Messungen für die zumindest eine gemessene Größe durch die verstrichene Zeit während der Bestrahlung bestimmt.

So kann die Messung der jeweiligen Größe etwa jeweils nach Ablauf eines festen Intervalls durchgeführt werden; am besten so schnell aufeinanderfolgend wie möglich, also mit einem möglichst kurzen Intervall. Die Zeitpunkte der Messungen sind bei dieser Vorgehensweise entsprechend unabhängig vom Anfahren der Zielpunkte. Mit anderen Worten: Das Anfahren der Zielpunkte und die Messungen der Strahlposition und/oder der Strahlintensität erfolgen asynchron. Für die Positionsmessungen und die Intensitätsmessungen können etwa auch unterschiedliche Intervalle gewählt werden; in einem solchen Fall sind auch die Positionsmessungen und die Intensitätsmessungen asynchron zueinander.

Vorteilhaft kann es auch sein, die Bestrahlung über die Intensität des Strahls zu steuern und die Position des Strahls wiederholt jeweils nach einem festen Zeitintervall zumessen. Auch in diesem Fall sind die Messungen der beiden Größen asynchron zueinander und die Messung der Position des Strahls asynchron zum Anfahren der Zielpunkte.

Um eine bestimmte Dosis in einem bestimmten Zielpunkt mittels Zeitsteuerung - also bei einem durch die verstrichene Zeit bestimmten Anfahren der Zielpunkte - zu applizieren, wird für den Strahl eine Scangeschwindigkeit gewählt, die erwarten lässt, dass die beabsichtigte Dosis an diesem Punkt auch appliziert wird. Verweilt der Strahl an einem Zielpunkt, so wird er auf den nächsten Zielpunkt gerichtet, sobald eine Zeit abgelaufen ist, die erwarten lässt, dass die beabsichtigte Dosis appliziert wurde.

So eine Zeitsteuerung schließt es jedoch nicht aus, Kenntnisse über Eigenschaften des Strahls mit einzubeziehen. So ist es bevorzugt, das Extraktionsprofil der Strahlenquelle zu berücksichtigen. Die Anzahl der extrahierten Partikel kann nämlich, je nach Quelle, stark mit der Zeit variieren. Das Extraktionsprofil für eine bestimmte Quelle ist i.d.R. bekannt; es kann etwa im Vorfeld der Bestrahlung bestimmt werden. Für den einfachen Fall einer homogenen Solldosisverteilung bedeutet dies, dass die Scangeschwindigkeit vergleichsweise hoch gewählt wird, wenn nach dem Extraktionsprofil die Partikelanzahl hoch ist, und die Scangeschwindigkeit vergleichsweise niedrig gewählt wird, wenn nach dem Extraktionsprofil die Partikelanzahl gering ist. Ist die Solldosisverteilung nicht homogen, so ist dies gewichtend mit einzubeziehen. Geht man ohne Beachtung des Extraktionsprofil von einer mittleren Strahlintensität und einer entsprechenden Scangeschwindigkeit aus, so ist im Vergleich dazu die Scangeschwindigkeit zu erhöhen, wenn nach dem Extraktionsprofil die Strahlintensität hoch ist, und die Scangeschwindigkeit zu verringern, wenn die Strahlintensität nach dem Extraktionsprofil gering ist. Abweichungen von der Solldosisverteilung können so verringert werden; die zu applizierende Teilchenzahl kann präziser eingehalten werden.

Einige Detektortypen, etwa Vieldraht-Proportionalkammern, können eine Positionsmessung schneller bei einem Strahl mit geringerem Querschnitt durchführen als bei einem Strahl mit größerem Querschnitt. Der Querschnitt des Strahls nimmt in Richtung Ziel zu. Daher ist es bevorzugt, die Strahlposition so weit in Richtung Quelle des Strahls zu messen, dass der Querschnitt des Strahls höchstens 80%, bevorzugter 60%, seines Querschnitts unmittelbar vor dem Ziel entspricht.

Vorzugsweise wird die Breite des Strahls mit einem eigenen Modul bestimmt. Im Vergleich zu einer Lösung, bei der die Breite des Strahls mit dem gleichen Modul bestimmt wird, mit dem auch die Position des Strahls bestimmt wird, kann ein Geschwindigkeitsvorteil erzielt werden. Ein Modul kann etwa einer vollständigen Auswertungs- und/oder Steuerkette vom Detektor bis zur Software entsprechen. Es kann sich bspw. auch um einen eigenen Rechner oder einen eigenen Prozess auf einem Rechner handeln, was ggf. schon reicht, um die genannten Größen getrennt voneinander zu ermitteln.

Die Erfindung richtet sich auch auf eine Vorrichtung zum Bestrahlen eines Ziels mit einem Zielpunkte anfahrenden Strahl mit einer Messeinrichtung zum Messen zumindest einer der Größen Position des Strahls und Intensität des Strahls und mit einer Ablaufsteuerung, die dazu ausgelegt ist, den Strahl in Abhängigkeit von der zumindest einen gemessenen Größe, insbesondere von einer Abweichung betreffend die zumindest eine gemessene Größe, zu ändern. Die Bestrahlungsvorrichtung ist dadurch gekennzeichnet, dass sie dazu ausgelegt ist, die zumindest eine gemessene Größe pro Zielpunkt höchstens einmal zu messen.

So eine Bestrahlungsvorrichtung umfasst auch eine Einrichtung zur Erzeugung eines Strahls, insbesondere zur Erzeugung eines Partikelstrahls beziehungsweise insbesondere eines Ionenstrahls. Als Strahlerzeugungseinrichtungen kommt etwa ein Beschleuniger, insbesondere ein Synchrotron oder ein Cyclotron in Betracht.

Wird mit einem Ionenstrahl bestrahlt, so umfasst die Bestrahlungsvorrichtung auch eine Scanning-Einrichtung, kurz einen Scanner, umfassend Scanning-Magnete zum Ablenken des Ionenstrahls und/oder auch einen Tiefenmodulator.

Zur Positionsmessung bietet sich eine MWPC, eine strip ionization chamber, eine Messkammer basierend auf Szintillatoren oder PET an. Die Intensität kann etwa mit einer Ionisationskammer (IC) oder auch mit einer MWPC, einer pixel ionization chamber oder ebenfalls mit einer Messkammer basierend auf Szintillatoren gemessen werden.

Ablaufsteuerungen für gattungsgemäße Bestrahlungsvorrichtungen sind grundsätzlich bekannt; beispielsweise ist eine in der oben genannten Publikation von Haberer et al. gezeigt. Aufgaben einer solchen Ablaufsteuerung umfassen typischerweise Maßnahmen betreffend die Steuerung des Ablaufs der Bestrahlung, insbesondere des Scannings, die maximale Dosis, einen Vergleich mit einem Bestrahlungsplan, und die Dokumentation.

Die Ablaufsteuerung greift gegebenenfalls auch in den Bestrahlungsablauf ein (Interlock), etwa durch eine Unterbrechung oder Korrektur desselben, beispielsweise wenn der Strahl in seiner Intensität schwankt, oder bei sonstigen Störungen.

Die Ablaufsteuerung kann etwa mit Hilfe eines Rechners oder eines Rechnersystems implementiert werden. So können in einem solchen Rechner etwa Informationen betreffend die Energieaufteilung der Durchgänge und der isoenergetischen Schichten, die Zielpunkte, die Rasterpunkte, die Zieldosis pro Raterpunkt, den Behandlungsplan und Kriterien für ein Beenden der Bestrahlung abgelegt sein. Ein Kriterium für das Beenden der Bestrahlung kann etwa das Erreichen der im Behandlungsplan festgelegten Zieldosis pro Zielpunkt sein; das Erreichen der Zieldosis kann an die Scanning-Einrichtung rückgekoppelt werden. Es bietet sich an, die Zieldosis pro Zielpunkt in einer Tabelle abzulegen. Eine entsprechende oder zumindest vergleichbare Tabelle kann auch für jeden Durchgang beim Rescanning angelegt werden, enthaltend alle Parameter für die Ablaufsteuerung.

Der Rechner kann mit mehreren Modulen für jeweils eine Funktion aufgebaut sein; das Rechnersystemen kann mit mehreren Rechnern für jeweils eine Funktion aufgebaut sein. Beispiele für solche Funktionen sind: Scannersteuerung, Pulszentrale (fordert eine Ionenstrahl an), Intensitätsmessung (zumindest eine, besser zwei pro Zielpunkt zur Erhöhung der Redundanz, soweit höchstens eine Positionsmessung durchgefiihrt wird), Positionsmessung (zumindest eine, besser zwei pro Zielpunkt zur Erhöhung der Redundanz, soweit höchstens eine Intensitätsmessung durchgeführt wird), Dokumentation; typischerweise werden die Strahlparameter aus der Pulszentrale, die Scannerparameter und die Ergebnisse der Intensitäts- und Positionsmessungen dokumentiert.

Sind Einrichtungen zur Intensitätsmessung und zur Positionsmessung mehrfach in der Vorrichtung vorhanden - dies kann zur Erhöhung der Redundanz wünschenswert sein - so ist es bevorzugt, diese in Serie zu schalten, um die Abtastrate der Messungen zu erhöhen, also die Dauer der Messungen insgesamt zu verringern. Weist die Vorrichtung etwa jeweils zwei Einrichtungen zur Intensitätsmessung und zur Positionsmessung auf und werden jeweils die Einrichtungen zu Intensitätsmessung und die Einrichtungen zu Positionsmessung in Serie geschaltet, kann die jeweils maximale Abtastrate gegebenenfalls verdoppelt werden. Bei diesen Einrichtungen kann es sich etwa um Detektoren, Module, Rechner oder allgm. Auswerteelektronik handeln.

Die Bestrahlungsvorrichtung ist vorzugsweise zur Ausführung des erfindungsgemäßen Verfahrens in einer beliebigen der bevorzugten Ausgestaltungen ausgelegt.

Die Erfindung richtet sich auch auf ein Kontrollsystem zur Steuerung der Bestrahlungsvorrichtung. Im Unterschied zu Bestrahlungsvorrichtung umfasst das Kontrollsystem selbst keine Strahlerzeugungsvorrichtung. Wird das Kontrollsystem zur Steuerung einer Bestrahlungsvorrichtung zur Bestrahlung von Menschen oder Tieren eingesetzt, so spricht man auch von einem Therapiekontrollsystem (TCS).

Das erfindungsgemäße Verfahren kann etwa durch eine Modifikation der Positionsmesseinrichtung, auch genannt Positionsmessmodul (SAM; Speicher- und Auslesemodul), welche Teil des Kontrollsystem sein kann, erfolgen. Dabei wird das Kontrollsystem um einen Baustein erweitert oder aber ein bestehendes Modul wird ersetzt. Grundsätzlich ist eine Anpassung der Ablaufkontrolle erforderlich; ggf. reicht eine Anpassung der Steuersoftware.

Die vorangehende und die folgende Beschreibung der einzelnen Merkmale bezieht sich sowohl auf das Verfahren als auch auf die Vorrichtung und das Kontrollsystem, ohne dass dies im Einzelnen in jedem Fall explizit erwähnt ist; die dabei offenbarten Einzelmerkmale können auch in anderen als den gezeigten Kombinationen erfindungswesentlich sein.

Im Folgenden soll die Erfindung auch anhand von Ausführungsbeispielen näher erläutert werden.
Fig. 1 zeigt eine schematische Darstellung einer erfindungsgemäßen Bestrahlungsvorrichtung mit einer erfindungsgemäßen Kontrollvorrichtung zur Durchführung erfindungsgemäßer Verfahren.
Fig. 2 zeigt ein Ablaufschema eines erfindungsgemäßen Verfahrens.
Fig. 3 zeigt eine Auswahl erfindungsgemäßer Maßnahmen betreffend die Messung von Strahlposition und Strahlintensität.
Fig. 4 zeigt eine Auswahl erfindungsgemäßer Maßnahmen betreffend Änderungen des Strahls.
Fig.5 zeigt ein schematisches Extraktionsprofil der Vorrichtung aus Fig. 1.

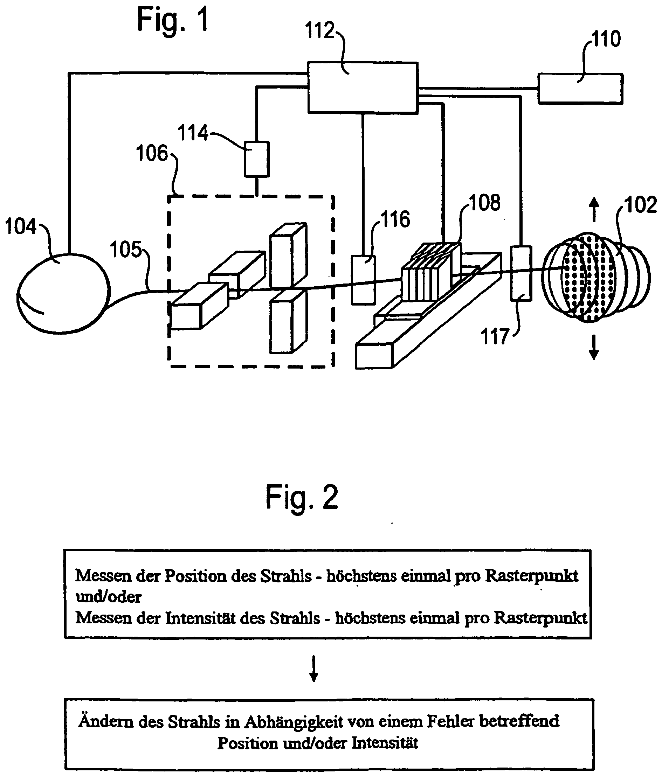

Die Bestrahlungsvorrichtung ist dazu ausgelegt, ein Zielvolumen 102 zu bestrahlen. Bei dem Zielvolumen 102 handelt es sich etwa um einen nahe bzw. in der Lunge einer Person gelegenen Tumor; das hier gezeigte Zielvolumen 102 hat senkrecht zur Strahlrichtung einen Durchmesser von ca. 20 cm. Alternativ kann es sich auch um ein Phantom, etwa basierend auf Wasser oder Plexiglas, oder ein sonstiges Material handeln. Das Zielvolumen bewegt sich zyklisch etwa auf und ab, was in Fig. 1 durch die Pfeile über und unter dem Zielvolumen 102 angedeutet ist.

Die Bestrahlungsvorrichtung weist ein Synchrotron, ein Cyclotron oder einen sonstigen Beschleuniger 104 zur Bereitstellung eines Partikelstrahls 105, bspw. bestehend aus Protonen oder ¹²C Kernen, auf. Typischerweise hat so ein Strahl eine Ausdehnung von einem oder mehreren Millimetern, etwa 3 bis 10 mm. In dem Zielvolumen 102 sind Schichten angedeutet, welche der Tiefe des Bragg-Peaks für eine bestimmte Teilchenenergie entsprechen (isoenergetische Schichten).

Als Scanning-Verfahren ist hier Rasterscanning gewählt. Mit dem Partikelstrahl 105 fährt die Bestrahlungsvorrichtung schematisch als schwarze Punkte in dem Zielvolumen 102 dargestellte Rasterpunkte an; diese liegen in dem Zielvolumen etwa 2 mm auseinander. Es ist, wegen der einfacheren Darstellung, schematisch ein schichtweises Anfahren von Rasterpunkten gezeigt; alternativ können Rasterpunkte natürlich auch volumetrisch angefahren werden (nicht dargestellt).

Eine laterale Beeinflussung des Partikelstrahls 105 kann mittels Scanning-Magneten 106 erfolgen. Hier handelt es sich um Dipolmagnete 106. Für eine longitudinale (entlang der Strahlrichtung) Beeinflussung weist die Bestrahlungsvorrichtung eine passive Energievariationseinrichtung, etwa in Form eines Keilsystems 108, zur Energiemodulation auf. Das Keilsystem 108 weist Keile etwa aus Kunststoff auf, die über einen linearen Motor (nicht gezeigt) bewegt werden können. Das Keilsystem 108 wird bevorzugt bei volumetrischen Scans verwendet. Bei schichtweisem Scanning so wird die Energie bevorzugt über den Beschleuniger oder eine vor den Scanning-Magneten 106 installierte Energiemodulationseinheit geändert.

Weiter weist die Bestrahlungsvorrichtung eine Ablaufsteuerung 112, eine Scannerkontrolle 114, einen Teilchenzähler 116 und einen Positionsdetektor 117 auf; Teilchenzähler 116 und Positionsdetektor 117 können auch an anderen Positionen untergebracht sein, etwa beide an der dargestellten Position des Teilchenzählers 116. Die Ablaufsteuerung 112 fungiert hier als Kontrollsystem.

Der Teilchenzähler 116 ist eine Ionisationskammer (IC) und fungiert hier als Intensitätsmesseinrichtung. Der Positionsdetektor 117 ist eine Vieldraht-Proportionalkammer (MWPC). Die Ionisationskammer 116 benötigt für eine Intensitätsmessung 15 µs. Die Vieldraht-Proportionalkammer 117 benötigte eine Bestimmung der Position des Strahls 105 150 µs. Die Position des Strahls kann etwa unmittelbar vor dem Ziel oder auch etwa 1 m vor dem Ziel gemessen werden. Alternativ bietet es sich an, die Position des Strahls weiter in Richtung Strahlenquelle zu messen, da dort der Strahlquerschnitt kleiner ist und daher die Positionsmessung schneller durchgeführt werden kann. Der Positionsdetektor 117 kann etwas so angeordnet werden, dass der Strahlquerschnitt höchstens 50% seines Wertes unmittelbar vor dem Ziel beträgt.

Es sind jeweils zwei Module zur Bestimmung der Intensität und zur Bestimmung der Position des Strahls vorgesehen. Diese sind jeweils in Serie geschaltet (nicht gezeigt). Hier ist es möglich, etwa durch "zeitversetztes" Messen eine höhere zeitliche Auflösung zu erzielen. Zur Erhöhung der Sicherheit kann dann durch einen die Zeitversetzung berücksichtigen Vergleich auch eine Redundanz zur Verfügung gestellt werden.

Der Teilchenzähler 116 bestimmt die Anzahl der Teilchen in dem Partikelstrahl 105 und führt das Ergebnis der Ablaufsteuerung 112 zu. Die Ablaufsteuerung 112 ist dazu ausgelegt, den Beschleuniger 104, die Scanning-Magneten 106 und das Keilsystem 108 zu steuern. Dazu ermittelt die Ablaufsteuerung 112 entsprechende Steuerungsparameter unter Berücksichtigung der von dem Teilchenzähler 116 und dem Positionsdetektor 117 empfangenen Daten.

Die Bestrahlung erfolgt in mehreren Durchgängen, es wird also Rescanning angewendet.

Es können etwa 5 oder 10, besser 15 bis 20 oder bis 30 Durchgänge gewählt werden. Typischerweise ist das Zielvolumen in 50 Schichten unterteilt. Es dauert typischerweise 100ms bis zu 1s, um eine Schicht vollständig anzufahren. Wird ein Volumen angefahren, liegt die Anfahrdauer typischerweise im Bereich von Sekunden.

Während der Bestrahlung wird grundsätzlich entweder höchstens eine Positionsmessung oder höchstens eine Intensitätsmessung jeweils pro Rasterpunkt durchgeführt, vgl. Fig. 2.

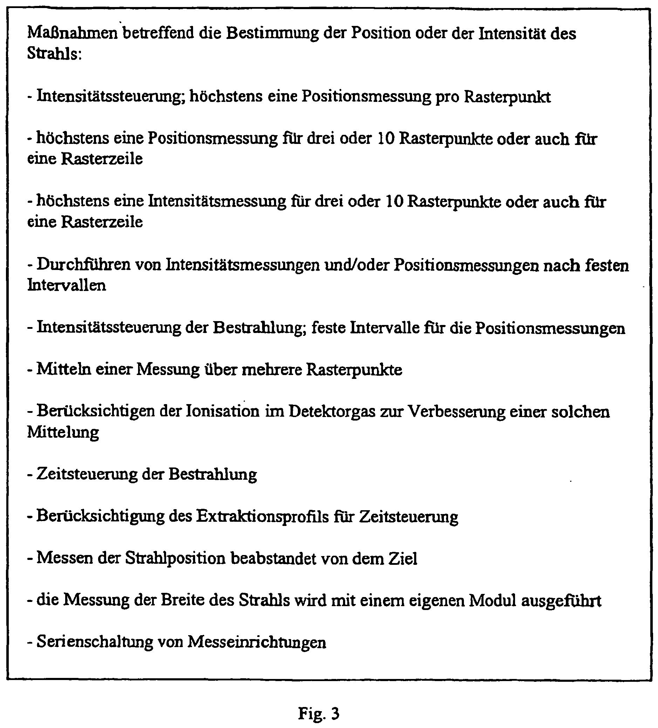

Mögliche Maßnahmen betreffend die Bestimmung der Position des Strahls oder der Intensität desselben nach der Erfindung sind etwa:
- höchstens eine Positionsmessung pro Rasterpunkt, keinerlei Intensitätsmessung;
- höchstens eine Intensitätsmessung pro Rasterpunkt, keinerlei Positionsmessung;
- höchstens eine Positionsmessung pro Rasterpunkt sowie höchstens eine Intensitätsmessung pro Rasterpunkt;
- höchstens eine Positionsmessung pro Rasterpunkt sowie mehrere Intensitätsmessungen pro Rasterpunkt;
- höchstens eine Intensitätsmessung pro Rasterpunkt sowie mehrere Positionsmessungen pro Rasterpunkt;
- Steuerung des Ablaufs der Bestrahlung nach der Intensität des Strahls (Intensitätssteuerung), gegebenenfalls mit mehreren Intensitätsmessungen pro Rasterpunkt, wobei die Position des Strahls höchstens einmal pro Rasterpunkt gemessen wird;
- höchstens eine Positionsmessung für drei oder 10 Rasterpunkte oder auch für eine Rasterzeile;
- höchstens eine Intensitätsmessung für drei oder 10 Rasterpunkte oder auch für eine Rasterzeile;
- Durchführen von Intensitätsmessungen und/oder Positionsmessungen nach festen Intervallen;
- Wahl der Intervalle so kurz wie möglich;
- Intensitätssteuerung der Bestrahlung; feste Intervalle für die Positionsmessungen;
- Auslegen der Intervalle, so dass drei oder 10 Rasterpunkte oder auch eine Rasterzeile zwischen den Messungen angefahren werden;
- Auslegen der Intervalle, so dass die Bestrahlungszeit nicht durch die Messungen verlängert wird;
- Mitteln einer Messung über mehrere Rasterpunkte;
- Berücksichtigen der Ionisation im Detektorgas zur Verbesserung einer solchen Mittelung;
- Zeitsteuerung der Bestrahlung, wobei die Positionen höchstens einmal pro Rasterpunkt gemessen wird;
- Berücksichtigung des Extraktionsprofils bei einer solchen Zeitsteuerung;
- Messen der Strahlposition relativ nahe an Strahlenquelle;
- die Messung der Breite des Strahls wird mit einem eigenem Modul, entkoppelt von der Ortmessung, ausgeführt;
- Serienschaltung von Messeinrichtungen;
eine Auswahl ist in Figur 3 gezeigt.

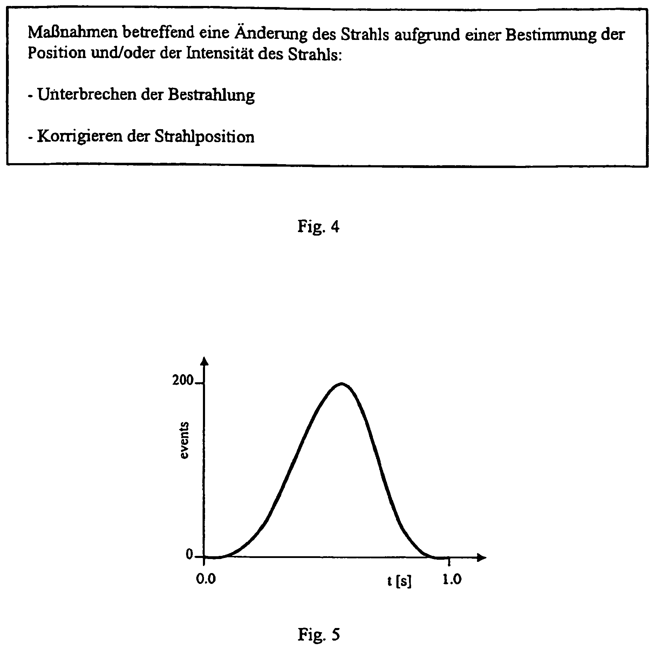

Mögliche Maßnahmen betreffend eine Änderung des Strahls aufgrund einer Bestimmung der Positionen oder der Intensität desselben nach der Erfindung sind etwa:
- Unterbrechen der Bestrahlung, falls die gemessene Position oder die gemessene Intensität erheblich von dem entsprechenden Soll abweicht;
- Korrigieren der Strahlposition, falls die gemessene Position geringfügig von ihrem Soll abweicht;
eine Auswahl ist in Figur 4 gezeigt.

Figur 5 zeigt ein schematisches Extraktionsprofil der in Figur 1 gezeigten Vorrichtung für einen Kryptonstrahl mit einer Energie von 300 MeV/u. Ein reales (Synchrotron-)Profil würde neben Anstieg und Abfall auch ein Plateau mit Fluktuationen, sog. "ripple", aufweisen. Man erkennt, dass die Partikelzahl (events) pro Extraktion (spill) zunächst schnell zunimmt, bei circa einer halben Sekunde ein Maximum durchläuft, um dann bei einer Sekunde schon im wesentlichen vollständig wieder auf Null abgefallen zu sein. Wird das Anfahren der Rasterpunkte nicht über die Intensität des Strahls gesteuert, sondern zeitlich, so wird dieses Extraktionsprofil zur Bestimmung der Scangeschwindigkeit berücksichtigt; ist die Anzahl extrahierten Partikel besonders hoch, so kann eine schnelle Scangeschwindigkeit gewählt werden, ist sie niedrig, so wird eine niedrige Scangeschwindigkeit gewählt.

## Patentansprüche

1. Verfahren zum Bestrahlen eines Ziels (102) aus organischem oder anorganischem Material bei dem die Bestrahlung von Personen oder Tieren ausgenommen ist mit einem Zielpunkte anfahrenden Strahl (105) mit den Schritten:
Messen zumindest einer der Größen Position des Strahles und Intensität des Strahls,
Ändern des Strahls in Abhängigkeit von der zumindest einen gemessenen Größe,
insbesondere von einer Abweichung betreffend die zumindest eine gemessene Größe,
**dadurch gekennzeichnet,**
**dass** pro Zielpunkt die zumindest eine gemessene Größe höchstens einmal gemessen wird.

2. Verfahren nach Anspruch 1, bei dem die Position des Strahls (105) höchstens einmal pro Zielpunkt gemessen wird.

3. Verfahren nach Anspruch 1 oder 2, bei dem die Bestrahlung als Mehrfachbestrahlung mit zumindest zwei Durchgängen ausgelegt ist, wobei in jedem der Durchgänge Zielpunkte aufeinanderfolgend angefahren werden.

4. Verfahren nach Anspruch 3, bei dem das Ziel (102) bewegt ist.

5. Verfahren nach einem der vorangehenden Ansprüche, bei dem die zumindest eine gemessene Größe höchstens einmal für zumindest zwei Zielpunkte gemessen wird.

6. Verfahren nach einem der vorangehenden Ansprüche, bei dem die Zeitpunkte zur Messung der zumindest einen gemessenen Größe durch die verstrichene Zeit bestimmt sind.

7. Verfahren nach einem der vorangehenden Ansprüche, bei dem das Anfahren der aufeinanderfolgenden Zielpunkte durch die bis dahin verstrichene Zeit bestimmt wird.

8. Verfahren nach Anspruch 7, bei dem das Anfahren der aufeinander folgenden Zielpunkte auch durch das Extraktionsprofil bestimmt ist.

9. Verfahren nach einem der vorangehenden Ansprüche, bei dem die Strahlposition so weit in Richtung Quelle des Strahls (104) gemessen wird, dass der Querschnitt des Strahls (105) höchstens 80% des Querschnitts des Strahls unmittelbar vor dem Ziel beträgt.

10. Verfahrens nach einem der vorangehenden Ansprüche, bei dem die Breite des Strahls (105) mit einem Strahlbreitenmessmodul gemessen wird.

11. Vorrichtung zum Bestrahlen eines Ziels (102) mit einem Zielpunkte anfahrenden Strahl (105) mit
einer Messeinrichtung (116, 117) zum Messen zumindest einer der Größen Position (117) des Strahls (105) und Intensität (116) des Strahls (105) und mit
einer Ablaufsteuerung (112), die dazu ausgelegt ist, den Strahl (105) in Abhängigkeit von der zumindest einen gemessenen Größe, insbesondere von einer Abweichung betreffend die zumindest eine gemessene Größe, zu ändern,
**dadurch gekennzeichnet, dass** die Vorrichtung dazu ausgelegt ist, die zumindest eine gemessene Größe pro Zielpunkt höchstens einmal zu messen.

12. Vorrichtung nach Anspruch 11, bei der mehrere Messeinrichtungen in Serie geschaltet sind.

13. Vorrichtung nach Anspruch 11 oder 12 ausgelegt zur Ausführung eines Verfahrens nach einem der Ansprüche 1 bis 10.

14. Kontrollsystem (112) ausgelegt zur Steuerung einer Vorrichtung nach einem der Ansprüche 11 bis 13 mit den Verfahrensschritten:
Messen zumindest einer der Größen Position des Strahls und Intensität des Strahls,
Ändern des Strahls in Abhängigkeit von der zumindest einen gemessenen Größe,
insbesondere von einer Abweichung betreffend die zumindest eine gemessene Größe,
**dadurch gekennzeichnet,**
**dass** pro Zielpunkt die zumindest eine gemessene Größe höchstens einmal gemessen wird.

15. Kontrollsystem (112) nach Anspruch 14, ferner mit zumindest einem weiteren Verfahrensschritt nach einem der Ansprüche 2 bis 10.

## Claims

1. Method for irradiating a target (102) of organic or inorganic material, wherein irradiation of individuals or animals is disclaimed, with a beam (105) approaching target points, involving the following steps:
Measuring at least one of the parameters relating to the position of the beam and the intensity of the beam,
changing the beam as a function of the at least one measured parameter,
particularly as a function of a variance relating to the at least one measured parameter,
**characterized in**
**that** the at least one measured parameter is measured at the most once per target point.

2. Method according to Claim 1, wherein the position of the beam (105) is measured at the most once per target point.

3. Method according to Claim 1 or 2, wherein the irradiation process is designed a multiple irradiation with at least two scans, wherein in each of the scans target points are approached successively.

4. Method according to Claim 3, wherein the target (102) is moved.

5. Method according to any one of the preceding claims, wherein the at least one measured parameter is measured at the most once for at least two target points.

6. Method according to any one of the preceding claims, wherein the points in time of the measurement of the at least one measured parameter is determined by the elapsed time.

7. Method according to any one of the preceding claims, wherein the approach of the successive target points is determined by the elapsed time.

8. Method according to Claim 7, wherein the approach of the successive target points is determined by the extraction profile.

9. Method according to any one of the preceding claims, wherein the beam position is measured as far in the direction of the source of the beam (104) that the cross-section of the beam (105) corresponds to at the most 80% of the cross-section of the beam directly before the target.

10. Method according to any one of the preceding claims, wherein the width of the beam (105) is measured with a beam width measuring module.

11. Device for irradiating a target (102) with a beam (105) approaching target points comprising
a measuring device (116, 117) for measuring at least one of the parameters relating to the position (117) of the beam (105) and the intensity (116) of the beam (105), and comprising
a sequence control system (112) designed to change the beam (105) as a function of the at least one measured parameter, particularly as a function of a variance relating to the at least one measured parameter, **characterized in that** the device is designed to measure the at least one measured parameter at the most once per target point.

12. Device according to Claim 11, wherein several measuring devices are connected in series.

13. Device according to Claim 11 or 12, designed to perform a method in accordance with claims 1 to 10.

14. Control system (112) designed for controlling a device in accordance with any one of claims 11 to 13 with the following method steps:
Measuring at least one of the parameters relating to the position of the beam and the intensity of the beam,
changing the beam as a function of the at least one measured parameter,
particularly as a function of a variance relating to the at least one measured parameter,
wherein the at least one measured parameter is measured at the most once per target point.

15. Control system (112) according to Claim 14, further comprising at least one further method step according to one of the claims 2 to 10.

## Revendications

1. Procédé d'irradiation d'une cible (102) composée d'un matériau organique ou inorganique, selon lequel l'irradiation de personnes ou d'animaux est exclue, avec un faisceau (105) visant des points cibles, comprenant les étapes suivantes :
mesure d'au moins une des grandeurs que sont la position du faisceau et l'intensité du faisceau,
modification du faisceau en fonction de la grandeur mesurée, au nombre d'au moins une,
en particulier d'un écart concernant la grandeur mesurée, au nombre d'au moins une,
**caractérisé en ce que** pour chaque point cible, la grandeur mesurée, au nombre d'au moins une, est mesurée au maximum une fois.

2. Procédé selon la revendication 1, selon lequel la position du faisceau (105) est mesurée au maximum une fois pour chaque point cible.

3. Procédé selon la revendication 1 ou 2, selon lequel l'irradiation est conçue comme irradiation multiple avec au moins deux passages, des points cibles étant visés successivement lors de chacun des passages.

4. Procédé selon la revendication 3, selon lequel la cible (102) est mobile.

5. Procédé selon l'une des revendications précédentes, selon lequel la grandeur mesurée, au nombre d'au moins une, est mesurée au maximum une fois pour au moins deux points cibles.

6. Procédé selon l'une des revendications précédentes, selon lequel les instants pour la mesure de la grandeur mesurée, au nombre d'au moins une, sont déterminés par le temps écoulé.

7. Procédé selon l'une des revendications précédentes, selon lequel la visée des points cibles successifs est déterminée par le temps écoulé jusque-là.

8. Procédé selon la revendication 7, selon lequel la visée des points cibles successifs est également déterminée par le profil d'extraction.

9. Procédé selon l'une des revendications précédentes, selon lequel la position de faisceau est mesurée en direction de la source du faisceau (104) sur une distance telle que la section transversale du faisceau (105) corresponde au maximum à 80 % de la section transversale du faisceau immédiatement avant la cible.

10. Procédé selon l'une des revendications précédentes, selon lequel la largeur du faisceau (105) est mesurée avec un module de mesure de largeur de faisceau.

11. Dispositif d'irradiation d'une cible (102) avec un faisceau (105) visant des points cibles, comprenant :
un dispositif de mesure (116, 117) pour la mesure d'au moins une des grandeurs que sont la position (117) du faisceau et l'intensité (116) du faisceau (105), et comprenant
une commande séquentielle (112) qui est conçue pour modifier le faisceau (105) en fonction de la grandeur mesurée, au nombre d'au moins une, en particulier d'un écart concernant la grandeur mesurée, au nombre d'au moins une,
**caractérisé en ce que** le dispositif est conçu pour mesurer au maximum une fois la grandeur mesurée, au nombre d'au moins une.

12. Dispositif selon la revendication 11, dans lequel plusieurs dispositifs de mesure sont montés en série.

13. Dispositif selon la revendication 11 ou 12, conçu pour la mise en oeuvre du procédé selon l'une des revendications 1 à 10.

14. Système de contrôle (112), conçu pour la commande d'un dispositif selon l'une des revendications 11 à 13, avec les étapes de procédé suivantes :
mesure d'au moins une des grandeurs que sont la position du faisceau et l'intensité du faisceau,
modification du faisceau en fonction de la grandeur mesurée, au nombre d'au moins une,
en particulier d'un écart concernant la grandeur mesurée, au nombre d'au moins une,
**caractérisé en ce que** pour chaque point cible, la grandeur mesurée, au nombre d'au moins une est mesurée au maximum une fois.

15. Système de contrôle (112) selon la revendication 14, comprenant en outre au moins une étape de procédé supplémentaire selon l'une des revendications 2 à 10.
